# EUROPÄISCHE PATENTSCHRIFT

(11) **EP 1 214 302 B1**
(45) Veröffentlichungstag und Bekanntmachung des Hinweises auf die Patenterteilung: **29.10.2003**
(21) Anmeldenummer: 00962346.3
(22) Anmeldetag: 22.08.2000
(51) Int. Cl.: C07D 271/113, C07D 413/12, A61K 31/4245, A61P 3/10

(54) **SUBSTITUIERTE 3-PHENYL-5-ALKOXI-1,3,4-OXDIAZOL-2-ONE UND IHRE VERWENDUNG ALS LIPASE-HEMMER**
SUBSTITUTED 3-PHENYL-5-ALKOXI-1,3,4-OXDIAZOL-2-ONES AND THEIR USE AS LIPASE INHIBITORS
3-PHENYL-5-ALCOXI-1,3,4-OXDIAZOL-2-ONES SUBSTITUEES, LEUR PRODUCTION ET LEUR UTILISATION COMME INHIBITEURS DE LIPASE

(30) Priorität: 04.09.1999 DE 19942354
(43) Veröffentlichungstag der Anmeldung: 19.06.2002
(73) Patentinhaber: Aventis Pharma Deutschland GmbH, 65929 Frankfurt am Main (DE)
(72) Erfinder: PETRY, Stefan, 65929 Frankfurt (DE); SCHOENAFINGER, Karl, 63755 Alzenau (DE); MUELLER, Guenter, 65843 Sulzbach (DE); BARINGHAUS, Karl-Heinz, 61200 Wölfersheim (DE)
(86) Internationale Anmeldenummer: EP0008150
(87) Internationale Veröffentlichungsnummer: WO01017981

(56) Entgegenhaltungen:
- WO-A-96/13264

## Beschreibung

Die Erfindung betrifft substituierte 3-Phenyl-5-alkoxi-1,3,4-oxdiazol-2-one, die eine hemmende Wirkung an der hormonsensitiven Lipase, HSL, zeigen.

Bestimmte 5-Alkoxi-1,3,4-oxdiazol-2-one mit einem ortho-substituierten Phenylring als Substituenten oder mit ankondensierten fünf- oder sechsgliedrigen Ringen besitzen anthelmintische (DE-A 26 04 110) und insektizide Wirkung (DE-A 26 03 877, EP-B 0 048 040, EP-B 0 067 471).

Bestimmte 5-Phenoxi-1,3,4-oxdiazol-2-one mit einem ortho-substituierten Phenylring als Substituenten zeigen endoparasitizide Wirkung (EP-A 0 419 918).

Ziel der Erfindung war es nun, Verbindungen zu finden, die eine hemmende Wirkung an der hormonsensitiven Lipase, HSL, zeigen.

Dies wurde erreicht mit den substituierten 3-Phenyl-5-alkoxi-1,3,4-oxdiazol-2-onen der allgemeinen Formel 1, 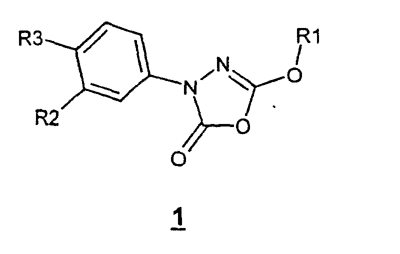 worin
- R¹: C₁-C₆-Alkyl, C₃-C₉-Cycloalkyl, wobei beide Gruppen gegebenenfalls durch Phenyl, das wiederum durch Halogen, C₁-C₄-Alkyl, O-C₁-C₄-Alkyl, Nitro, CF₃, ein- oder mehrfach substituiert sein kann, O-C₁-C₄-Alkyl, S-C₁-C₄-Alky, N(C₁-C₄-Alkyl)₂ ein- oder mehrfach substituiert sind und
- R² und R³: unabhängig voneinander Wasserstoff, C₆-C₁₀-Aryl, C₃-C₈-Cycloalkyl, gegebenenfalls durch C₁-C₄-Alkyl substituiertes C₆-C₁₀-Aryloximethyl, gegebenenfalls durch Halogen, CF₃ oder C₁-C₄-Alkyl ein- oder mehrfach substituiertes O-Benzyl, O-C₆-C₁₀-Aryl oder O-C₃-C₈-Cycloalkyl, ein- oder mehrfach durch Fluor, C₆-C₁₀-Aryl oder Amino substituiertes O-C₁-C₆-Alkyl, wobei Amino wiederum durch C₁-C₄-Alkyl ein- oder mehrfach substituiert sein kann, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, gegebenenfalls durch C₁-C₄-Alkyl ein- oder mehrfach substituiert, SO₂-N(C₁-C₆-Alkyl)₂ oder COX bedeuten,
wobei X für O-C₁-C₆-Alkyl, NH-C₁-C₆-Alkyl, NH-C₃-C₈-Cycloalkyl oder N(C₁-C₆-Alkyl)₂ steht, und
N(C₁-C₆-Alkyl)₂ auch für Pyrrolidino, Piperidino, Morpholino, Thiomorpholino oder Piperazino stehen kann, die gegebenenfalls durch C₁-C₄-Alkyl, Benzyl, C₆-C₁₀-Aryl, CO-C₁-C₄-Alkyl, CO-C₆-C₁₀-Aryl, CO-O-C₁-C₄-Alkyl, SO₂-C₁-C₄-Alkyl oder SO₂-C₆-C₁₀-Aryl substituiert sein können,
mit der Maßgabe, dass R² und R³ nicht gleichzeitig Wasserstoff bedeuten sowie deren physiologisch verträgliche Salze und optische Isomere.

Die erwähnten Arylreste können gegebenenfalls mit C₁-C₄-Alkyl, C₁-C₄-Alkoxi, Halogen, Trifluormethyl ein- oder mehrfach substituiert sein. Die erwähnten Cycloalkylreste können gegebenenfalls mit C₁-C₄-Alkyl ein- oder mehrfach substituiert sein und die erwähnten Alkylreste können durch Hydroxi, Di-C₁-C₄alkylamino und Fluor substituiert sein. Halogen steht für Fluor, Chlor, Brom, bevorzugt für Fluor und Chlor.

Bevorzugt sind substituierte 3-Phenyl-5-alkoxi-1,3,4-oxdiazol-2-one der Formel 1, worin einer der Reste R² oder R³ Wasserstoff bedeutet.

Besonders bevorzugt sind substituierte 3-Phenyl-5-alkoxi-1,3,4-oxdiazol-2-one der Formel 1, worin
- R¹: C₁-C₆-Alkyl, das gegebenenfalls durch Phenyl substituiert sein kann, bedeutet.

Besonders bevorzugt sind ferner substituierte 3-Phenyl-5-alkoxi-1,3,4-oxdiazol-2-one der Formel 1, worin
- R² und R³: unabhängig voneinander Wasserstoff, C₆-C₁₀-Aryl, C₃-C₈-Cycloalkyl, gegebenenfalls durch C₁-C₄-Alkyl substituiertes C₆-C₁₀-Aryloximethyl, gegebenenfalls durch C₁-C₄-Alkyl oder Halogen ein- oder mehrfach substituiertes O-Benzyl O-C₆-C₁₀-Aryl oder O-C₃-C₈-Cycloalkyl, ein- oder mehrfach durch Fluor, C₆-C₁₀-Aryl oder Amino substituiertes O-C₁-C₆-Alkyl,
wobei Amino wiederum durch C₁-C₄-Alkyl ein- oder mehrfach substituiert sein kann, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, substituiert durch C₁-C₄-Alkyl, SO₂-N(C₁-C₆-Alkyl)₂ oder CO-N(C₁-C₆-Alkyl)₂ bedeuten, und
N(C₁-C₆-Alkyl)₂ auch für Piperidino, Morpholino oder Piperazino stehen kann, die gegebenenfalls durch C₁-C₄-Alkyl substituiert sein können.

Ganz besonders bevorzugt sind auch substituierte 3-Phenyl-5-alkoxi-1,3,4-oxdiazol-2-one der Formel 1, worin
- R²: Wasserstoff, C₆-C₁₀-Aryl, O-C₆-C₁₀-Aryl, gegebenenfalls durch C₁-C₄-Alkyl substituiertes C₆-C₁₀-Aryloximethyl, O-Benzyl, ein- oder mehrfach durch Fluor oder Amino substituiertes O-C₁-C₆-Alkyl, wobei Amino wiederum durch C₁-C₄-Alkyl ein- oder mehrfach substituiert sein kann, gegebenenfalls durch C₁-C₄-Alkyl ein- oder mehrfach substituiertes O-C₃-C₈-Cycloalkyl und
- R³: Wasserstoff, C₆-C₁₀-Aryl, C₃-C₈-Cycloalkyl, gegebenenfalls durch C₁-C₄-Alkyl oder Halogen ein- oder mehrfach substituiertes O-C₆-C₁₀-Aryl oder O-C₃-C₈-Cycloalkyl, ein- oder mehrfach durch Fluor substituiertes O-C₁-C₆-Alkyl, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, ein- oder mehrfach substituiert durch C₁-C₄-Alkyl, SO₂-N(C₁-C₆-Alkyl)₂ oder CO-N(C₁-C₆-Alkyl)₂ bedeuten, und
N(C₁-C₆-Alkyl)₂ auch für Piperidino, Morpholino oder Piperazino steht, die gegebenenfalls durch C₁-C₄-Alkyl substituiert sein können. Ganz besonders bevorzugt sind substituierte 3-Phenyl-5-alkoxi-1,3,4-oxdiazol-2-one der Formel 1, worin
- R¹: für Methyl, Ethyl, Butyl, Isopropyl oder Benzyl steht und
- R²: für Wasserstoff, Trifluormethoxi, Trifluorbutoxi, 3,3,5,5-Tetramethylcyclohexyloxi, Benzyloxi, Phenoxi, Phenyl, 2-Diethylaminoethyloxi oder 3-Methylphenoxi-methyl, und
- R³: für Wasserstoff, Trifluormethoxi, 3,3,5,5-Tetramethylcyclohexyloxi, Phenoxi, 4-Chlorphenoxi, Cyclohexyl, Phenyl, Morpholinosulfonyl, 3,3,5-Trimethylcyclohexyl-aminosulfonyl, 2,2,6,6,-Tetramethyl-piperidin-4-ylaminosulfonyl, 2-(Diisopropylaminoethyl)amino-sulfonyl, 4-Methyl-piperazin-1-yl-sulfonyl, 3,3,-Dimethylpiperidino-carbonyl oder 3,5-Dichlorphenoxi stehen.

Ganz besonders bevorzugt sind ferner substituierte 3-Phenyl-5-alkoxi-1,3,4-oxdiazol-2-one der Formel 1, worin
- R¹: für Methyl, Ethyl, Butyl, Isopropyl oder Benzyl steht und
- R²: für Wasserstoff, Trifluormethoxi, 3,3,5,5-Tetramethylcyclohexyloxi, Benzyloxi oder Phenoxi und
- R³: für Wasserstoff, Trifluormethoxi, 3,3,5,5-Tetramethylcyclohexyloxi, Phenoxi, Cyclohexyl, Phenyl, Morpholinosulfonyl oder 3,3,5-Trimethylcyclohexylaminosulfonyl stehen.

Die erfindungsgemäßen Verbindungen der allgemeinen Formel 1 besitzen eine überraschende hemmende Wirkung an der hormonsensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.

Die Herstellung der erfindungsgemäßen Verbindungen der allgemeinen Formel 1 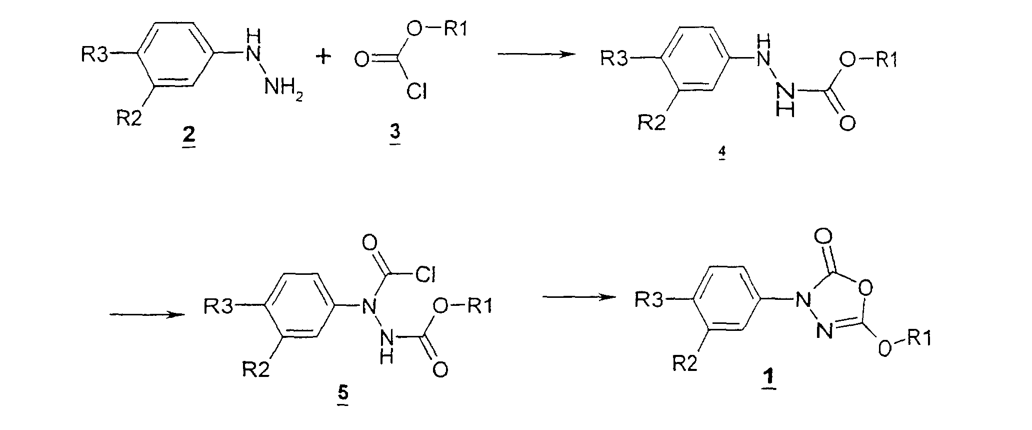 kann nach an und für sich bekannten Methoden auf verschiedenen Wegen erfolgen. Beispielsweise kann die Herstellung der substituierten 3-Phenyl-5-alkoxi-1,3,4-oxdiazol-2-one der allgemeinen Formel 1 erfolgen, durch Umsetzung von Hydrazinen der allgemeinen Formel 2 mit Chlorameisensäureestern der Formel 3 oder anderen reaktiven Kohlensäureesterderivaten, worin R¹, R² und R³ wie oben definiert sind, zu den Verbindungen der Formel 4, welche mit Phosgen, Carbonyldiimidazol oder Diphosgen acyliert und zyklisiert werden zu den Verbindungen der Formel 1. Da bei diesen Reaktionen in der Regel Säuren freigesetzt werden, empfiehlt es sich zur Beschleunigung Basen wie Pyridin, Triethylamin, Natronlauge oder Alkalicarbonate zuzusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, nHeptan, Dioxan, Diethylether zu Einsatz.

Die Hydrazine der Formel 2 sind kommerziell erhältlich oder lassen sich nach bekannten Methoden zB. durch Diazotierung der entsprechenden Aniline mit 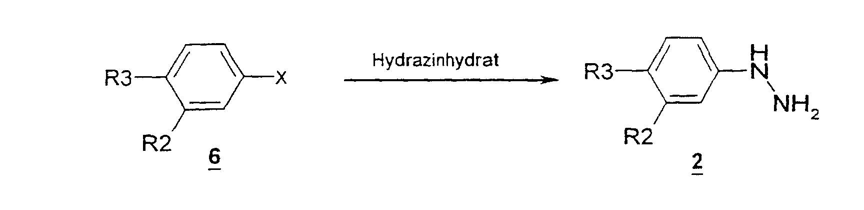 nachfolgender Reduktion oder durch nukleophile Substitution von entsprechend substituierten Phenylderivaten 6 (X= F, Cl, Br, I, OSO₂CF₃) mit Hydrazinhydrat herstellen. Solche geeignete Phenylderivate können Nitro substituierte Halogenbenzole, vorzugsweise Fluor- und Chlor-nitrobenzole sein, aus denen sich an geeigneter Stelle im Syntheseweg durch Reduktion und Umsetzung mit Acylierungs- oder Alkylierungsmitteln ,wie beispielweise Säurechloriden, Anhydriden, Isocyanaten, Chlorameisensäureestern, Sulfonsäurechloriden oder Alkyl- und Arylalkyl-halogeniden, die erfindungsgemäßen Verbindungen herstellen lassen.

Die Wirkung der erfindungsgemäßen Verbindungen der Formel 1 wurde an folgendem Enzymtestsytem geprüft:

### Enzympräparation:

Präparation der partiell gereinigten HSL:
Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml
   Antipain, 20 µg/ml Pepstatin) gewaschen und schließlich in 10 ml
   Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall
SS34, 12000 rpm, 10 min, 4°C) gesammelt und in 2.5 ml 20 mM Tris/HCl, pH 7.0, 1 mM EDTA, 65 mM NaCI, 13 % sucrose, 1 mM DTT, 10 µg/ml
Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay:

Für die Herstellung des Substrats werden 25-50 µCi [³H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N₂ getrocknet und dann in 2 ml 0.1 M KPᵢ (pH 7.0) durch Ultraschallbehandlung (Branson 250, Mikrospitze, Einstellung 1-2, 2 x 1 min im 1-min Intervall) aufgenommen. Nach Zugabe von 1 ml KPᵢ und erneuter Ultraschallbehandlung (4 x 30 sec auf Eis in 30-sec Intervallen) wird 1 ml 20% BSA (in KPᵢ) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPᵢ, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K₂CO₃, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT , benutzt.

In diesem Test zeigten die Verbindungen die folgende Wirkung:

| Verbindung Beispiel Nr. | IC₅₀ (µM) |
|---|---|
| 8 | 15 |
| 10 | 6 |
| 11 | 10 |
| 12 | 10 |
| 13 | 10 |
| 14 | 50 |
| 15 | 2 |
| 16 | 2 |
| 17 | 2 |
| 18 | <1 |
| 19 | 5 |
| 33 | 4 |
| 34 | 3 |
| 35 | 3 |
| 36 | 2 |
| 37 | 3 |
| 38 | <1 |
| 39 | 60 |

Die nachfolgenden Beispiele erläutern die Herstellungsmethoden näher, ohne sie einzuschränken.

### Beispiele:

### Beispiel 1:

### 4-Fluor-benzolsulfonsäure-morpholid (Zwischenprodukt)

Zur eisgekühlten Lösung von 19,5 g 4-Fluor-benzolsulfonsäure-chlorid in 100 ml Toluol wurden 20 g Morpholin getropft und die Mischung 1 Stunde am Rückfluß erhitzt. Nach dem Erkalten wurde im Vakuum eingeengt, mit Wasser verrührt, der Niederschlag abgesaugt, mit Wasser gewaschen und aus Isopropanol umkristallisiert.
Ausbeute:16,9 g Fp.: 140°C

### Beispiel 2:

### 4-Hydrazino-benzolsulfonsäure-morpholid (Zwischenprodukt)

5 g 4-Fluor-benzolsulfonsäure-morpholid wurden in 15 ml N-Methylpyrrolidon gelöst, mit 2,5 g Hydrazinhydrat versetzt und 1 Stunde auf 100°C erhitzt. Nach Abkühlung auf Raumtemperatur wurden 75 ml Wasser zugefügt und bei Raumtemperatur gerührt. Nach 2 Stunden wurde der Feststoff abgesaugt und aus Isopropanol umkristallisiert.
Ausbeute: 3,2 g Fp.: 164°C

Analog wurde das folgende Beispiel hergestellt:

### Beispiel 3:

### 4-Hydrazino-benzolsulfonsäure-(3,3,5-trimethyl-cyclohexyl)-amid (Zwischenprodukt) Fp.: 129°C

### Beispiel 4:

### 4-(3,3,5,5-Tetramethylcyclohexyl-oxi)nitrobenzol (Zwischenprodukt)

Zur Lösung von 7,8 g 3,3,5,5-Tetramethylcyclohexanol in 50 ml Dimethylformamid werden 1,3 g Natriumhydrid gefügt und die Mischung 30 Min bei 40-50°C gerührt. Dann werden portionsweise insgesamt 7,0 g 4-Fluor-nitrobenzol zugefügt und die Mischung anschließend 3 Stunden auf 100°C erhitzt und auf Raumtemperatur abgekühlt. Nach Zusatz von 250 ml Eiswasser wird verrührt und der gebildete Feststoff abgesaugt und im Vakuum getrocknet..
Ausbeute: 8,6 g Fp.: 70°C

### Beispiel 5:

### 4-(3,3,5,5-Tetramethylcyclohexyl-oxi)-anilin (Zwischenprodukt)

8,3 g 4-(3,3,5,5-Tetramethylcyclohexyloxi)-nitrobenzol werden in 500 ml Methanol in Gegenwart von 400 mg Platindioxid unter Normaldruck bis zum Ende der Wasserstoffaufnahme hydriert. Nach dem Abfiltrieren des Katalysators wird die Lösung einrotiert und der Rückstand, ein allmählich erstarrendes, bräunliches Öl, ohne weitere Reinigung für die weiteren Umsetzungen verwendet.
Ausbeute: 7,3 g

### Beispiel 6:

### 4-(3,3,5,5-Tetramethylcyclohexyl-oxi)-phenylhydrazin-hydrochlorid (Zwischenprodukt)

Zur auf -10°C abgekühlten, gerührten Mischung bestehend aus 3,7 g 4-(3,3,5,5-Tetramethylcyclohexyl-oxi)-anilin, 7,5 ml Wasser und 15,5 ml konz. HCl wird die Lösung von 1,13 g Natriumnitrit in 7,5 ml Wasser zugetropft und die Mischung bei-10°C 45 Min nachgerührt und anschließend zur Suspension von 9,3 g Zinndichloriddihydrat in 7 ml konz. HCI zugetropft. Der Niederschlag wird abgesaugt, mit Wasser gewaschen, unter Stickstoff in 200 ml Wasser aufgeschlämmt und mit 100 ml 30%ige Natronlauge bei 10-15°C zersetzt. Der erneut sich bildende Niederschlag wird abgesaugt, mit Wasser gewaschen, in 200 ml Ether aufgenommen und mit Natriumsulfat getrocknet. Dann wird das Produkt mit etherischer HCI gefällt, abgesaugt und im Vakuum getrocknet.
Ausbeute: 2,1 g Fp.: 171°C

### Beispiel 7:

### N'-(4-Morpholinosulfonyl-phenyl)-hydrazino-ameisensäureethylester (Zwischenprodukt)

Zur Mischung bestehend aus 0,275 g 4-Hydrazino-benzolsulfonsäure-morpholid, 5 ml Methylenchlorid und 1 ml Pyridin wurden vorsichtig unter Eiskühlung 114 mg Chlorameisensäureethylester zugetropft und dann unter langsamen Erwärmen auf RT gerührt. Nach Verdünnen mit 10 ml Wasser wurde das Produkt mit Essigester ausgeschüttelt, die Essigesterphase mehrmals mit Wasser gewaschen, über Natriumsulfat getrocknet und eingeengt. Das so erhaltene ölige Rohprodukt wurde ohne weitere Reinigung weiter umgesetzt.
Ausbeute: 0,25 g

### Beispiel 8:

### 3-(4-Morpholinosulfonyl-phenyl)-5-ethoxi-1,3,4-oxdiazol-2-on

Das Öl aus Beispiel 3 wurde in 5 ml Methylenchlorid aufgenommen und unter Umrühren und Eiskühlung mit 1 ml einer 20%igen toluolischen Phosgenlösung versetzt. Diese Mischung stand über Nacht bei Raumtemperatur und wurde mit weiteren 10 ml Methylenchlorid verdünnt und dann 3 mal mit Wasser gewaschen. Nach dem Trocknen über Natriumsulfat wurde die Mischung im Vakuum eingeengt und das Produkt durch Säulenchromatografie (Kieselgel, Lösungsmittel: Methanol:Methylenchlorid = 2 : 98) gereinigt.
Ausbeute:130 mg Fp.: 195°C

Die folgenden Beispiele wurden analog Beispiel 4 hergestellt:

### Beispiel 9:

### 3-(4-Morpholinosulfonyl-phenyl)-5-methoxi-1,3,4-oxdiazol-2-on

Fp.: 164°C

### Beispiel 10:

### 3-(4-Trifluormethoxi-phenyl)-5-methoxi-1,3,4-oxdiazol-2-on

Fp.: 52°C

### Beispiel 11:

### 3-(4-Trifluormethoxi-phenyl)-5-ethoxi-1,3,4-oxdiazol-2-on

Fp.: 63°C

### Beispiel 12:

### 3-(4-Trifluormethoxi-phenyl)-5-isopropoxi-1,3,4-oxdiazol-2-on

Fp.: Öl

### Beispiel 13:

### 3-(4-Trifluormethoxi-phenyl)-5-butoxi-1,3,4-oxdiazol-2-on

Fp.: Öl

### Beispiel 14:

### 3-(4-Trifluormethoxi-phenyl)-5-benzyloxi-1,3,4-oxdiazol-2-on

Fp.: Öl

### Beispiel 15:

### 3-(4-(3,3,5-Trimethyl-cyclohexyl-amino-sulfonyl)-phenyl)-5-methoxi-1,3,4-oxdiazol-2-on

Fp.: 164°C

### Beispiel 16:

### 3-(4-(3,3,5,5-Tetramethylcyclohexyl-oxi)-phenyl)-5-ethoxi-1,3,4-oxd i azol-2-on

Fp.: 111°C

### Beispiel 17:

### 3-(3-Benzyloxi-phenyl)-5-methoxi-1,3,4-oxdiazol-2-on

Fp.: Öl

### Beispiel 18:

### 3-(3-Benzyloxi-phenyl)-5-ethoxi-1,3,4-oxdiazol-2-on

Fp.: 85°C

### Beispiel 19:

### 3-(3-Trifluormethoxi-phenyl)-5-ethoxi-1,3,4-oxdiazol-2-on

Fp.: Öl

### Beispiel 20:

### 3-(3-Trifluormethoxi-phenyl)-5-methoxi-1,3,4-oxdiazol-2-on

Fp.: Öl

### Beispiel 21:

### 3-(3-Trifluormethoxi-phenyl)-5-isopropoxi-1,3,4-axdiazol-2-on

Fp.: Öl

### Beispiel 22:

### 3-(4-(2,2,6,6-Tetramethyl-piperidin-4-yl-amino-sulfonyl)-phenyl)-5-methoxi-1,3,4-oxdiazol-2-on

Fp.: Harz

### Beispiel 23:

### 3-(4-(2,2,6,6-Tetramethyl-piperidin-4-yl-amino-sulfonyl)-phenyl)-5-isopropoxi-1,3,4-oxdiazol-2-on

Fp.: Harz

### Beispiel 24:

### 3-(4-(2-(Diisopropylaminoethyl)amino-sulfonyl)-phenyl)-5-methoxi-1,3,4-oxdiazol-2-on

Fp.: Öl

### Beispiel 25:

### 3-(4-(2-(Diisopropylaminoethyl)amino-sulfonyl)-phenyl)-5-isopropoxi-1,3,4-oxdiazol-2-on

Fp.: Öl

### Beispiel 26:

### 3-(4-(4-Methyl-piperazin-1-yl-sulfonyl)-phenyl)-5-isopropoxi-1,3,4-oxdiazol-2-on

Fp.: Harz

### Beispiel 27:

### 3-(4-(4-Methyl-piperazin-1-yl-sulfonyl)-phenyl)-5-methoxi-1,3,4-oxdiazol-2-on

Fp.: Harz

### Beispiel 28:

### 3-(3-(4,4,4-Trifluor-butyl-oxi)-phenyl)-5-ethoxi-1,3,4-oxdiazol-2-on

Fp.: Öl

### Beispiel 29:

### 3-(3-(2-Diethylamino-ethyl-oxi)-phenyl)-5-ethoxi-1,3,4-oxdiazol-2-on

Fp.: Harz

### Beispiel 30:

### 3-(4-(4-Chlorphenoxi)-phenyl)-5-methoxi-1,3,4-oxdiazol-2-on

Fp.: 68°C

### Beispiel 31:

### 3-(4-(4-Chlorphenoxi)-phenyl)-5-isopropoxi-1,3,4-oxdiazol-2-on

Fp.: Öl

### Beispiel 32:

### 3-(4-(3,3,5-Trimethyl-cyclohexyl-amino-sulfonyl)-phenyl)-5-isopropoxi-1,3,4-oxdiazol-2-on

Fp.: Öl

### Beispiel 33:

### 3-(3-Phenoxi-phenyl)-5-methoxi-1,3,4-oxdiazol-2-on

Fp.: 89°C

### Beispiel 34:

### 3-(3-Phenoxi-phenyl)-5-ethoxi-1,3,4-oxdiazol-2-on

Fp.: 50°C

### Beispiel 35:

### 3-(3-Phenoxi-phenyl)-5-isopropoxi-1,3,4-oxdiazol-2-on

Fp.: 58°C

### Beispiel 36:

### 3-(4-Phenoxi-phenyl)-5-methoxi-1,3,4-oxdiazol-2-on

Fp.: 83°C

### Beispiel 37:

### 3-(4-Cyclohexyl-phenyl)-5-methoxi-1,3,4-oxdiazol-2-on

Fp.: Harz

### Beispiel 38:

### 3-(3-(3,3,5,5-Tetramethyl-cyclohexyl-oxi)-phenyl)-5-methoxi-1,3,4-oxdiazol-2-on

Fp.: 68°C

### Beispiel 39:

### 3-(4-Phenyl-phenyl)-5-methoxi-1,3,4-oxdiazol-2-on

Fp.: >260°C (Zers.)

### Beispiel 40:

### 3-(3-(3-Methylphenoxi-methyl)-phenyl)-5-methoxi-1,3,4-oxdiazol-2-on

Fp.: 47°C

### Beispiel 41:

### 3-(3-Phenyl-phenyl)-5-methoxi-1,3,4-oxdiazol-2-on

Fp.: 80°C

### Beispiel 42:

### 3-(4-(3,3-Dimethylpiperidino-carbonyl)-phenyl)-5-methoxi-1,3,4-oxdiazol-2-on

Fp.: Harz

### Beispiel 43:

### 3-(4-(3,3,5,5-Tetramethyl-cyclohexyloxi)-phenyl)-5-isopropoxi-1,3,4-oxdiazol-2-on

Fp.: Harz

## Patentansprüche

1. Substituierte 3-Phenyl-5-alkoxy-1,3,4-oxadiazol-2-one der allgemeinen Formel 1, 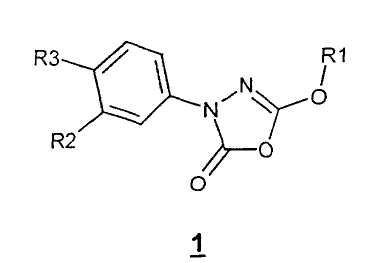 worin
R¹ C₁-C₆-Alkyl, C₃-C₉-Cycloalkyl, wobei beide Gruppen gegebenenfalls durch Phenyl, das wiederum durch Halogen, C₁-C₄-Alkyl, O-C₁-C₄-Alkyl, Nitro, CF₃, ein- oder mehrfach substituiert sein kann, O-C₁-C₄-Alkyl, S-C₁-C₄-Alkyl, N(C₁-C₄-Alkyl)₂ ein- oder mehrfach substituiert sind und
R² und R³ unabhängig voneinander Wasserstoff, C₆-C₁₀-Aryl, C₃-C₈-Cycloalkyl, gegebenenfalls durch C₁-C₄-Alkyl substituiertes C₆-C₁₀-Aryloximethyl, gegebenenfalls durch Halogen, CF₃ oder C₁-C₄-Alkyl ein- oder mehrfach substituiertes O-Benzyl, O-C₆-C₁₀-Aryl oder O-C₃-C₈-Cycloalkyl, ein- oder mehrfach durch Fluor, C₆-C₁₀-Aryl oder Amino substituiertes O-C₁-C₆-Alkyl, wobei Amino wiederum durch C₁-C₄-Alkyl ein- oder mehrfach substituiert sein kann, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, gegebenenfalls durch C₁-C₄-Alkyl ein- oder mehrfach substituiert, SO₂-N(C₁-C₆-Alkyl)₂ oder COX bedeuten,
wobei X für O-C₁-C₆-Alkyl, NH-C₁-C₆-Alkyl, NH-C₃-C₈-Cycloalkyl oder N(C₁-C₆-Alkyl)₂ steht, und N(C₁-C₆-Alkyl)₂ auch für Pyrrolidino, Piperidino, Morpholino, Thiomorpholino oder Piperazino stehen kann, die gegebenenfalls durch C₁-C₄-Alkyl, Benzyl, C₆-C₁₀-Aryl, CO-C₁-C₄-Alkyl, CO-C₆-C₁₀-Aryl, CO-O-C₁-C₄-Alkyl, SO₂-C₁-C₄-Alkyl oder SO₂-C₆-C₁₀-Aryl substituiert sein können,
mit der Maßgabe, dass R² und R³ nicht gleichzeitig Wasserstoff bedeuten sowie deren physiologisch verträgliche Salze und optische Isomere.

2. Substituierte 3-Phenyl-5-alkoxi-1,3,4-oxdiazol-2-one der Formel 1 gemäß Anspruch 1, worin einer der Reste R² oder R³ Wasserstoff bedeutet.

3. Substituierte 3-Phenyl-5-alkoxi-1,3,4-oxdiazol-2-one der Formel 1 gemäß den Ansprüchen 1 bis 2, worin
R¹ C₁-C₆-Alkyl, das gegebenenfalls durch Phenyl substituiert sein kann, bedeutet.

4. Substituierte 3-Phenyl-5-alkoxi-1,3,4-oxdiazol-2-one der Formel 1 gemäß den Ansprüchen 1 bis 3, worin
R² und R³ unabhängig voneinander Wasserstoff, C₆-C₁₀-Aryl, C₃-C₈-Cycloalkyl, gegebenenfalls durch C₁-C₄-Alkyl substituiertes C₆-C₁₀-Aryloximethyl, gegebenenfalls durch C₁-C₄-Alkyl oder Halogen ein- oder mehrfach substituiertes O-Benzyl O-C₆-C₁₀-Aryl oder O-C₃-C₈-Cycloalkyl, ein- oder mehrfach durch Fluor, C₆-C₁₀-Aryl oder Amino substituiertes O-C₁-C₆-Alkyl,
wobei Amino wiederum durch C₁-C₄-Alkyl ein- oder mehrfach substituiert sein kann, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, substituiert durch C₁-C₄-Alkyl, SO₂-N(C₁-C₆-Alkyl)₂ oder CO-N(C₁-C₆-Alkyl)₂ bedeuten, und
N(C₁-C₆-Alkyl)₂ auch für Piperidino, Morpholino oder Piperazino stehen kann, die gegebenenfalls durch C₁-C₄-Alkyl substituiert sein können.

5. Substituierte 3-Phenyl-5-alkoxi-1,3,4-oxdiazol-2-one der Formel 1 gemäß den Ansprüchen 1 bis 4, worin
R² Wasserstoff, C₆-C₁₀-Aryl, O-C₆-C₁₀-Aryl, gegebenenfalls durch C₁-C₄-Alkyl substituiertes C₆-C₁₀-Aryloximethyl, O-Benzyl, ein- oder mehrfach durch Fluor oder Amino substituiertes O-C₁-C₆-Alkyl, wobei Amino wiederum durch C₁-C₄-Alkyl ein- oder mehrfach substituiert sein kann, gegebenenfalls durch C₁-C₄-Alkyl ein- oder mehrfach substituiertes O-C₃-C₈-Cycloalkyl und
R³ Wasserstoff, C₆-C₁₀-Aryl, C₃-C₈-Cycloalkyl, gegebenenfalls durch C₁-C₄-Alkyl oder Halogen ein- oder mehrfach substituiertes O-C₆-C₁₀-Aryl oder O-C₃-C₈-Cycloalkyl, ein- oder mehrfach durch Fluor substituiertes O-C₁-C₆-Alkyl, SO₂-NH-C₁-C₆-Alkyl, gegebenenfalls durch N(C₁-C₆-Alkyl)₂ substituiert, SO₂-NH-(2,2,6,6-tetramethylpiperidin-4-yl), SO₂-NH-C₃-C₈-Cycloalkyl, ein- oder mehrfach substituiert durch C₁-C₄-Alkyl, SO₂-N(C₁-C₆-Alkyl)₂ oder CO-N(C₁-C₆-Alkyl)₂ bedeuten, und
N(C₁-C₆-Alkyl)₂ auch für Piperidino, Morpholino oder Piperazino steht, die gegebenenfalls durch C₁-C₄-Alkyl substituiert sein können.

6. Substituierte 3-Phenyl-5-alkoxi-1,3,4-oxdiazol-2-one der Formel 1 gemäß den Ansprüchen 1 bis 5, worin
R¹ für Methyl, Ethyl, Butyl, Isopropyl oder Benzyl steht und
R² für Wasserstoff, Trifluormethoxi, Trifluorbutoxi, 3,3,5,5-Tetramethylcyclohexyloxi, Benzyloxi, Phenoxi, Phenyl, 2-Diethylaminoethyloxi oder 3-Methylphenoxi-methyl, und
R³ für Wasserstoff, Trifluormethoxi, 3,3,5,5-Tetramethylcyclohexyloxi, Phenoxi, 4-Chlorphenoxi, Cyclohexyl, Phenyl, Morpholinosulfonyl, 3,3,5-Trimethylcyclohexyl-aminosulfonyl, 2,2,6,6,-Tetramethyl-piperidin-4-ylaminosulfonyl, 2-(Diisopropylaminoethyl)amino-sulfonyl, 4-Methyl-piperazin-1-yl-sulfonyl, 3,3,-Dimethylpiperidino-carbonyl oder 3,5-Dichlorphenoxi stehen.

7. Substituierte 3-Phenyl-5-alkoxi-1,3,4-oxdiazol-2-one der Formel 1 gemäß den Ansprüchen 1 bis 6, worin
R¹ für Methyl, Ethyl, Butyl, Isopropyl oder Benzyl steht und
R² für Wasserstoff, Trifluormethoxi, 3,3,5,5-Tetramethylcyclohexyloxi, Benzyloxi oder Phenoxi und
R³ für Wasserstoff, Trifluormethoxi, 3,3,5,5-Tetramethylcyclohexyloxi, Phenoxi, Cyclohexyl, Phenyl, Morpholinosulfonyl oder 3,3,5-Trimethylcyclohexylaminosulfonyl stehen.

8. Verfahren zur Herstellung der substituierten 3-Phenyl-5-alkoxi-1,3,4-oxdiazol-2-one der Formel 1 gemäß den Ansprüchen 1 bis 7, **gekennzeichnet durch** 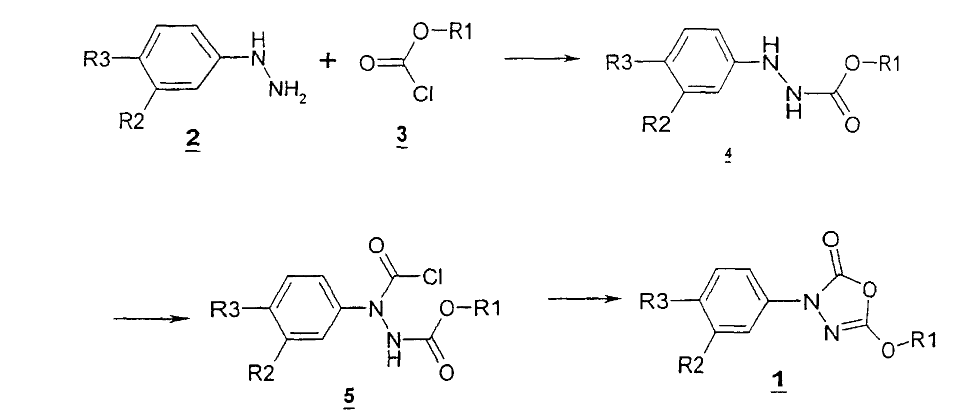 Umsetzung von Hydrazinen der allgemeinen Formel 2 mit Chlorameisensäureestern der Formel 3 oder anderen reaktiven Kohlensäureesterderivaten, worin R¹, R² und R³ wie in den Ansprüchen 1 bis 7 definiert sind, zu den Verbindungen der Formel 4, welche mit Phosgen, Carbonyldiimidazol oder Diphosgen acyliert und zyklisiert werden zu den Verbindungen der Formel 1.

9. Substituierte 3-Phenyl-5-alkoxi-1,3,4-oxdiazol-2-one der Formel 1 gemäß den Ansprüchen 1 bis 7 mit hemmender Wirkung der hormonsensitiven Lipase HSL.

10. Arzneimittel enthaltend ein substituiertes 3-Phenyl-5-alkoxi-1,3,4-oxdiazol-2-on der Formel 1 gemäß den Ansprüchen 1 bis 7.

11. Verwendung der substituierten 3-Phenyl-5-alkoxi-1,3,4-oxdiazol-2-one der Formel 1 gemäß den Ansprüchen 1 bis 7 zur Herstellung eines Heilmittels.

## Claims

1. A substituted 3-phenyl-5-alkoxy-1,3,4-oxadiazol-2-one of the formula 1, 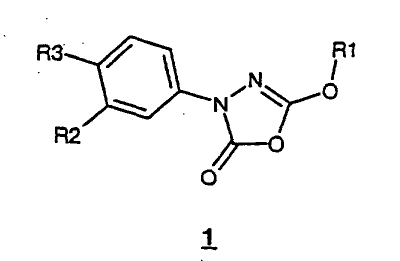 in which
R¹ is C₁-C₆-alkyl or C₃-C₉-cycloalkyl, where both groups are unsubstituted or mono- or polysubstituted by O-C₁-C₄-alkyl, S-C₁-C₄-alkyl, N(C₁-C₄-alkyl)₂ and/or phenyl which for its part may be mono- or polysubstituted by halogen, C₁-C₄-alkyl, O-C₁-C₄-alkyl, nitro, CF₃, and
R² and R³ independently of one another are hydrogen; C₆-C₁₀-aryl; C₃-C₈-cycloalkyl; optionally C₁-C₄-alkyl-substituted C₆-C₁₀-aryloxymethyl; O-C₃-C₈-cycloalkyl, O-C₆-C₁₀-aryl or O-benzyl, unsubstituted or mono- or polysubstituted by halogen, CF₃ or C₁-C₄-alkyl; O-C₁-C₆-alkyl which is mono- or polysubstituted by fluorine, C₆-C₁₀-aryl or amino, where amino for its part may be mono- or polysubstituted by C₁-C₄-alkyl; are SO₂-NH-C₁-C₆-alkyl, unsubstituted or substituted -by N(C₁-C₆-alkyl)₂; are SO₂-NH-(2,2,6,6-tetramethylpiperidin-4-yl); are SO₂-NH-C₃-C₈-cycloalkyl, unsubstituted or mono- or polysubstituted by C₁-C₄-alkyl; are SO₂-N(C₁-C₆-alkyl)₂ or COX,
where X is O-C₁-C₆-alkyl, NH-C₁-C₆-alkyl, NH-C₃-C₈-cycloalkyl or N(C₁-C₆-alkyl)₂, and
N(C₁-C₆-alkyl)₂ may also be pyrrolidino, piperidino, morpholino, thiomorpholino or piperazino which may be unsubstituted or substituted by C₁-C₄-alkyl, benzyl, C₆-C₁₀-aryl, CO-C₁-C₄-alkyl, CO-C₆-C₁₀-aryl, CO-O-C₁-C₄-alkyl, SO₂-C₁-C₄-alkyl or SO₂-C₆-C₁₀-aryl,
with the proviso that R² and R³ are not simultaneously hydrogen, and its physiologically acceptable salts and optical isomers.

2. The substituted 3-phenyl-5-alkoxy-1,3,4-oxadiazol-2-one of the formula 1 as claimed in claim 1 in which one of the radicals R² or R³ is hydrogen.

3. The substituted 3-phenyl-5-alkoxy-1,3,4-oxadiazol-2-one of the formula 1 as claimed in claim 1 or 2 in which
R¹ is C₁-C₆-alkyl which may be unsubstituted or substituted by phenyl.

4. The substituted 3-phenyl-5-alkoxy-1,3,4-oxadiazol-2-one of the formula 1 as claimed in any of claims 1 to 3 in which
R² and R³ independently of one another are hydrogen; C₆-C₁₀-aryl; C₃-C₈-cycloalkyl; unsubstituted or C₁-C₄-alkyl-substituted C₆-C₁₀-aryloxymethyl; O-C₃-C₈-cycloalkyl, O-C₆-C₁₀-aryl or O-benzyl, unsubstituted or mono- or polysubstituted by C₁-C₄-alkyl or halogen; O-C₁-C₆-alkyl which is mono- or polysubstituted by fluorine, C₆-C₁₀-aryl or amino, where amino for its part may be mono- or polysubstituted by C₁-C₄-alkyl; are SO₂-NH-C₁-C₆-alkyl, optionally substituted by N(C₁-C₆-alkyl)₂; are SO₂-NH-(2,2,6,6-tetramethylpiperidin-4-yl); are SO₂-NH-C₃-C₈-cycloalkyl, substituted by C₁-C₄-alkyl; are SO₂-N(C₁-C₆-alkyl)₂ or CO-N(C₁-C₆-alkyl)₂, and N(C₁-C₆-alkyl)₂ may also be piperidino, morpholino or piperazino which can be unsubstituted or substituted by C₁-C₄-alkyl.

5. The substituted 3-phenyl-5-alkoxy-1,3,4-oxadiazol-2-one of the formula 1 as claimed in any of claims 1 to 4 in which
R² is hydrogen; C₆-C₁₀-aryl; O-C₆-C₁₀-aryl; unsubstituted or C₁-C₄-alkyl-substituted C₆-C₁₀-aryloxymethyl, O-benzyl; is O-C₁-C₆-alkyl which is mono- or polysubstituted by fluorine or amino, where amino for its part may be mono- or polysubstituted by C₁-C₄-alkyl; is O-C₃-C₈-cycloalkyl which is unsubstituted or mono- or polysubstituted by C₁-C₄-alkyl; and
R³ is hydrogen; C₆-C₁₀-aryl; C₃-C₈-cycloalkyl; O-C₃-C₈-cycloalkyl or O-C₆-C₁₀-aryl which are unsubstituted or mono- or polysubstituted by C₁-C₄-alkyl or halogen; is O-C₁-C₆-alkyl which is mono- or polysubstituted by fluorine; is SO₂-NH-C₁-C₆-alkyl, unsubstituted or substituted by N(C₁-C₆-alkyl)₂; is SO₂-NH-(2,2,6,6-tetramethylpiperidin-4-yl); is SO₂-NH-C₃-C₈-cycloalkyl, mono- or polysubstituted by C₁-C₄-alkyl; is SO₂-N(C₁-C₆-alkyl)₂ or CO-N(C₁-C₆-alkyl)₂, and
N(C₁-C₆-alkyl)₂ is also piperidino, morpholino or piperazino, which may be unsubstituted or substituted by C₁-C₄-alkyl.

6. The substituted 3-phenyl-5-alkoxy-1,3,4-oxadiazol-2-one of the formula 1 as claimed in any of claims 1 to 5 in which
R¹ is methyl, ethyl, butyl, isopropyl or benzyl and
R² is hydrogen, trifluoromethoxy, trifluorobutoxy, 3,3,5,5-tetramethylcyclohexyloxy, benzyloxy, phenoxy, phenyl, 2-diethylaminoethyloxy or 3-methylphenoxymethyl, and
R³ is hydrogen, trifluoromethoxy, 3,3,5,5-tetramethylcyclohexyloxy, phenoxy, 4-chlorophenoxy, cyclohexyl, phenyl, morpholinosulfonyl, 3,3,5-trimethylcyclohexylaminosulfonyl, 2,2,6,6-tetramethylpiperidin-4-yl-aminosulfonyl, 2-(diisopropylaminoethyl)aminosulfonyl, 4-methylpiperazin-1-yl-sulfonyl, 3,3-dimethylpiperidinocarbonyl or 3,5-dichlorophenoxy.

7. The substituted 3-phenyl-5-alkoxy-1,3,4-oxadiazol-2-one of the formula 1 as claimed in any of claims 1 to 6 in which
R¹ is methyl, ethyl, butyl, isopropyl or benzyl and
R² is hydrogen, trifluoromethoxy, 3,3,5,5-tetramethylcyclohexyloxy, benzyloxy or phenoxy and
R³ is hydrogen, trifluoromethoxy, 3,3,5,5-tetramethylcyclohexyloxy, phenoxy, cyclohexyl, phenyl, morpholinosulfonyl or 3,3,5-trimethylcyclohexylaminosulfonyl.

8. A process for preparing the substituted 3-phenyl-5-alkoxy-1,3,4-oxadiazol-2-one of the formula 1 as claimed in any of claims 1 to 7, which comprises 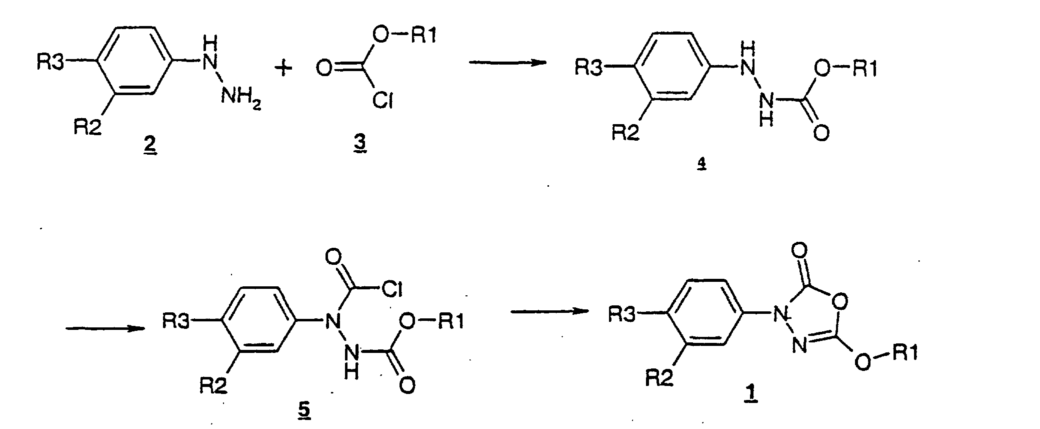 reacting hydrazines of the formula 2 with chloroformic esters of the formula 3 or other reactive carbonic acid ester derivatives, in which R¹, R² and R³ are as defined in any of claims 1 to 7, to give compounds of the formula 4 which are acylated with phosgene, carbonyldiimidazole or diphosgene and cyclized to give the compounds of the formula 1.

9. The substituted 3-phenyl-5-alkoxy-1,3,4-oxadiazol-2-one of the formula 1 as claimed in any of claims 1 to 7 having an inhibiting effect on the hormone-sensitive lipase HSL.

10. A medicament, comprising a substituted 3-phenyl-5-alkoxy-1,3,4-oxadiazol-2-one of the formula 1 as claimed in any of claims 1 to 7.

11. The use of the substituted 3-phenyl-5-alkoxy-1,3,4-oxadiazol-2-one of the formula 1 as claimed in any of claims 1 to 7 for preparing a therapeutic.

## Revendications

1. 3-phényl-5-alcoxy-1,3,4-oxadiazol-2-ones substituées de formule générale 1 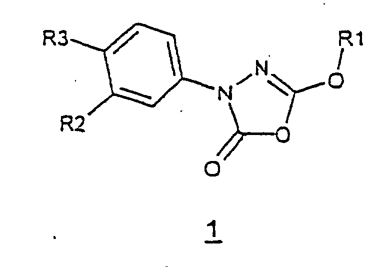 où
R¹ représente alkyle en C₁-C₆, cycloalkyle en C₃-C₉, où les deux groupes sont éventuellement substitués une ou plusiers fois par O-alkyle en C₁-C₄, S-alkyle en C₁-C₄, N(alkyle en C₁-C₄)₂, phényle où phényle peut être lui-même substitué une ou plusieurs fois par halogène, alkyle en C₁-C₄, O-alkyle en C₁-C₄, nitro, CF₃;
O-alkyle en C₁-C₄, S-alkyle en C₁-C₄, N(alkyle en C₁-C₄)₂, et
R² et R³ représentent indépendamment l'un de l'autre l'hydrogène; aryle en C₆-C₁₀; cycloalkyle en C₃-C₈; aryle en C₆-C₁₀-oxyméthyle éventuellement substitué par alkyle en C₁-C₄; O-benzyle, 0-aryle en C₆-C₁₀ ou 0-cycloalkyle en C₃-C₈ éventuellement substitué une ou plusieurs fois par halogène, CF₃ ou alkyle en C₁-C₄; O-alkyle en C₁-C₆ substitué une ou plusieurs fois par fluor, aryle en C₆-C₁₀ ou amino, où amino peut être lui-même substitué une ou plusieurs fois par alkyle en C₁-C₄;
SO₂-NH-alkyle en C₁-C₆, éventuellement substitué par N(alkyle en C₁-C₆)₂; SO₂-NH-(2,2,6,6-tétraméthylpipéridin-4-yle); SO₂-NH-cycloalkyle en C₃-C₈, éventuellement substitué une ou plusieurs fois par alkyle en C₁-C₄; SO₂-N(alkyle en C₁-C₆)₂ ou COX,
où X représente O-alkyle en C₁-C₆; NH-alkyle en C₁-C₆; NH-cycloalkyle en C₃-C₈ ou N(alkyle en C₁-C₆)₂, et
N(alkyle en C₁-C₆)₂ peut représenter aussi pyrrolidino, pipéridino, morpholino, thiomorpholino ou pipérazino, qui peuvent éventuellement être substitués par alkyle en C₁-C₄, benzyle, aryle en C₆-C₁₀, CO-alkyle en C₁-C₄, CO-aryle en C₆-C₁₀, CO-O-alkyle en C₁-C₄, SO₂-alkyle en C₁-C₄ ou SO₂-aryle en C₆-C₁₀;
à condition que R² et R³ ne représentent pas en même temps l'hydrogène, ainsi que leurs sels et isomères optiques physiologiquement acceptables.

2. 3-phényl-5-alcoxy-1,3,4-oxadiazol-2-ones substituées de formule 1 selon la revendication 1 où l'un des restes R² et R³ représente l'hydrogène.

3. 3-phényl-5-alcoxy-1,3,4-oxadiazol-2-ones substituées de formule 1 selon les revendications 1 à 2 où
R¹ représente alkyle en C₁-C₆ qui peut éventuellement être substitué par phényle.

4. 3-phényl-5-alcoxy-1,3,4-oxadiazol-2-ones substituées de formule 1 selon les revendications 1 à 3 où
R² et R³ représentent indépendamment l'un de l'autre l'hydrogène; aryle en C₆-C₁₀; cycloalkyle en C₃-C₈; aryle en C₆-C₁₀-oxyméthyle éventuellement substitué par alkyle en C₁-C₄; O-benzyle, O-aryle en C₆-C₁₀ ou O-cycloalkyle en C₃-C₈ éventuellement substitué une ou plusieurs fois par alkyle en C₁-C₄ ou halogène; O-alkyle en C₁-C₆ substitué une ou plusieurs fois par fluor, aryle en C₆-C₁₀ ou amino, où amino peut être lui-même substitué une ou plusieurs fois par alkyle en C₁-C₄;
SO₂-NH-alkyle en C₁-C₆, éventuellement substitué par N(alkyle en C₁-C₆)₂; SO₂-NH-(2,2,6,6-tétraméthylpipéridin-4-yle); SO₂-NH-cycloalkyle en C₃-C₈, substitué par alkyle en C₁-C₄; SO₂-N(alkyle en C₁-C₆)₂ ou CO-N(alkyle en C₁-C₆)₂ ; et
N(alkyle en C₁-C₆)₂ peut représenter aussi pipéridino, morpholino ou pipérazino, qui peuvent éventuellement être substitués par alkyle en C₁-C₄.

5. 3-phényl-5-alcoxy-1,3,4-oxadiazol-2-ones substituées de formule 1 selon les revendications 1 à 4 où
R² représente l'hydrogène; aryle en C₆-C₁₀; 0-aryle en C₆-C₁₀; aryle en C₆-C₁₀-oxyméthyle éventuellement substitué par alkyle en C₁-C₄; O-benzyle; O-alkyle en C₁-C₆ substitué une ou plusieurs fois par fluor ou amino, où amino peut être lui-même substitué une ou plusieurs fois par alkyle en C₁-C₄; O-cycloalkyle en C₃-C₈ éventuellement substitué une ou plusieurs fois par alkyle en C₁-C₄; et
R³ représente l'hydrogène; aryle en C₆-C₁₀; cycloalkyle en C₃-C₈; 0-aryle en C₆-C₁₀ ou O-cycloalkyle en C₃-C₈ éventuellement substitué une ou plusieurs fois par alkyle en C₁-C₄ ou halogène; O-alkyle en C₁-C₆ substitué une ou plusieurs fois par fluor;
SO₂-NH-alkyle en C₁-C₆, éventuellement substitué par N(alkyle en C₁-C₆)₂; SO₂-NH-(2,2,6,6-tétraméthylpipéridin-4-yle); SO₂-NH-cycloalkyle en C₃-C₈, substitué une ou plusieurs fois par alkyle en C₁-C₄; SO₂-N(alkyle en C₁-C₆)₂ ou CO-N(alkyle en C₁-C₆)₂; et
N(alkyle en C₁-C₆)₂ représente aussi pipéridino, morpholino, ou pipérazino, qui peuvent éventuellement être substitués par alkyle en C₁-C₄.

6. 3-phényl-5-alcoxy-1,3,4-oxadiazol-2-ones substituées de formule 1 selon les revendications 1 à 5 où
R¹ représente méthyle, éthyle, butyle, isopropyle ou benzyle et
R² représente l'hydrogène, trifluorométhoxy, trifluorobutoxy, 3,3,5,5-tétraméthylcyclohexyloxy, benzyloxy, phénoxy, phényle, 2-diéthylamino-éthyloxy ou 3-méthylphénoxyméthyle, et
R³ représente l'hydrogène, trifluorométhoxy, 3,3,5,5-tétraméthylcyclohexyloxy, phénoxy, 4-chlorophénoxy, cyclohexyle, phényle, morpholinosulfonyle, 3,3,5-triméthylcyclohexyl-aminosulfonyle, 2,2,6,6-tétraméthylpipéridin-4-yl-aminosulfonyle, 2-(diisopropylaminoéthyl)aminosulfonyle, 4-méthyl-pipérazin-1-yl-sulfonyle, 3,3-diméthylpipéridino-carbonyle ou 3,5-dichlorophénoxy.

7. 3-phényl-5-alcoxy-1,3,4-oxadiazol-2-ones substituées de formule 1 selon les revendications 1 à 6 où
R¹ représente méthyle, éthyle, butyle, isopropyle ou benzyle et
R² représente l'hydrogène, trifluorométhoxy, 3,3,5,5-tétraméthylcyclohexyloxy, benzyloxy ou phénoxy et
R³ représente l'hydrogène, trifluorométhoxy, 3,3,5,5-tétraméthylcyclohexyloxy, phénoxy, cyclohexyle, phényle, morpholinosulfonyle ou 3,3,5-triméthylcyclohexyl-aminosulfonyle.

8. Procédé de préparation des 3-phényl-5-alcoxy-1,3,4-oxadiazol-2-ones substituées de formule 1 selon les revendications 1 à 7 **caractérisé par** 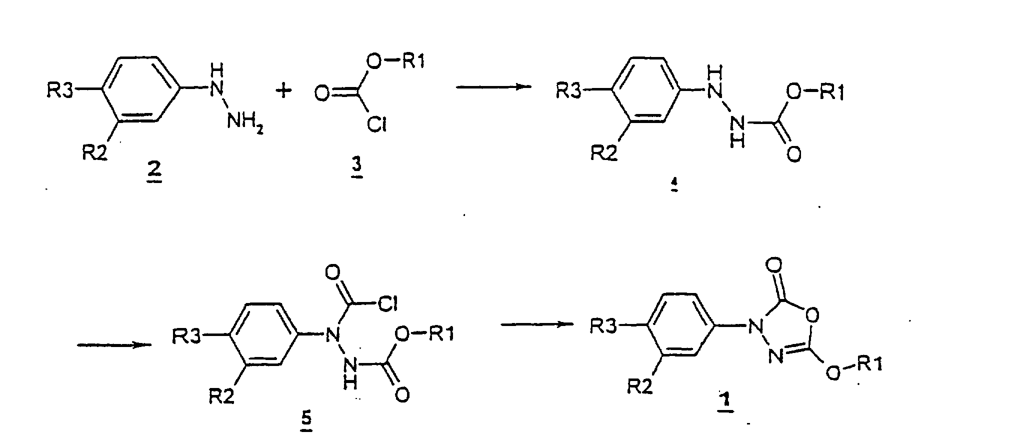 la réaction d'hydrazines de formule générale 2 avec des esters d'acide chloroformique de formule 3 ou d'autres dérivés d'esters d'acide carbonique réactifs, où R¹, R² et R³ sont définis comme dans les revendications 1 à 7, en les composés de formule 4, qui sont acylés et cyclisés en les composés de formule 1 avec le phosgène, le carbonyldiimidazole ou le diphosgène.

9. 3-phényl-5-alcoxy-1,3,4-oxadiazol-2-ones substituées de formule 1 selon les revendications 1 à 7 à effet inhibiteur de la lipase sensible aux hormones HSL.

10. Médicament contenant une 3-phényl-5-alcoxy-1,3,4-oxadiazol-2-one substituée de formule 1 selon les revendications 1 à 7.

11. Utilisation des 3-phényl-5-alcoxy-1,3,4-oxadiazol-2-ones substituées de formule 1 selon les revendications 1 à 7 pour la production d'un médicament.
